Europäisches Patentamt

European Patent Office

Office européen des brevets

(19)

(11) Veröffentlichungsnummer: **0 122 574**
**B2**

(12) # NEUE EUROPÄISCHE PATENTSCHRIFT

(45) Veröffentlichungstag der neuen Patentschrift:
**10.10.90**

(21) Anmeldenummer: **84103930.8**

(22) Anmeldetag: **09.04.84**

(51) Int. Cl.⁵: **A 61 K 9/22,** A 61 K 9/26,
A 61 K 31/415

(54) Teilbare Tablette mit verzögerter Wirkstofffreigabe und Verfahren zu deren Herstellung.

(30) Priorität: **18.04.83 DE 3314003**

(43) Veröffentlichungstag der Anmeldung:
**24.10.84 Patentblatt 84/43**

(45) Bekanntmachung des Hinweises auf die
Patenterteilung:
**29.10.86 Patenblatt 86/44**

(45) Bekanntmachung des Hinweises auf die
Entscheidung über den Einspruch:
**10.10.90 Patentblatt 90/41**

(84) Benannte Vertragsstaaten:
**AT BE CH DE FR IT LI LU NL SE**

(56) Entgegenhaltungen:
**DE-A-1 123 437**
**DE-A-2 328 580**
**DE-A-3 030 622**
**US-A-2 987 445**
**US-A-4 361 546**

**Pharmazeutische Industrie, Band 32, Heft 6,**
**1970, S. 469-473**
**Pharma International, Heft 3, 1971,**
**Sonderdruck, S. 1-16**
**Produktinformation der Fa. Röhm Pharma**
**GmbH zu EUDRAGIT (R) E 30 D**

(73) Patentinhaber: **BOEHRINGER INGELHEIM KG**
**Postfach 200**
**D-6507 Ingelheim am Rhein (DE)**

(72) Erfinder: **Zierenberg, Bernd, Dr.**
**Veit-Stoss-Strasse 4**
**D-6507 Ingelheim/Rhein (DE)**
Erfinder: **Gupte, Arun Rajaram, Dr. Ing.**
**Oestricher Strasse 17**
**D-6507 Ingelheim/Rhein (DE)**

EP 0 122 574 B2

## Beschreibung

Die Erfindung betrifft eine teilbare Tablette mit kontrollierter und verzögerter Wirkstofffreigabe auf Polyacrylatbasis sowie ein Verfahren zu deren Herstellung.

Es ist bekannt, feste Arzneimittelzubereitungen herzustellen, die über längere Zeit eine im gesamten Gastrointestinaltrakt gleichbleibende Wirkstofffreisetzung gewährleisten und auf diese Weise für eine konstante Wirkstoffkonzentration im Körper sorgen. Diese Depotformen gestatten es, die Anzahl der täglich zu verabreichenden Arzneimittelgaben zu verringern und das Therapieschema wesentlich zu vereinfachen Üblicherweise werden als Depotformen Tabletten und Kapseln eingesetzt, die mit einem die Wirkstofffreigabe regulierenden Überzug versehen sind. Es ist auch bereits vorgeschlagen worden, Depotformen aus einem Granulatgemisch zu fertigen, dessen einzelne Anteile den Arzneimittelwirkstoff unterschiedlich schnell abgeben.

Darüber hinaus sind Tabletten mit Bruchrillen bekannt, die es ermoglichen, die Darreichungsformen in Teildosen zu unterteilen, um speziellen Therapieanforderungen gerecht zu werden. Solche teilbaren Tabletten müssen insbesondere der Forderung genügen, leicht und sicher teilbar sein und auch in den Bruchstücken eine exakte Dosierung gewährleisten.

Bei mit Überzügen versehenen Depottabletten ergibt sich der Nachteil, dass durch die Teilung die Gesamtoberfläche der Tablette entscheidend verändert, d.h. vergrössert wird und dass ein Teil des retardierenden Überzugs entfällt. Dadurch werden die Charakteristika der Wirkstofffreisetzung wesentlich geändert, so dass in vielen Fällen die Bruchstücke der teilbaren Depottablette die Eigenschaft der verzögerten und kontinuierlichen Wirkstofffreisetzung nicht mehr oder nur in sehr eingeschränktem Masse aufweisen.

Um diesen Nachteil zumindest teilweise zu beheben, wird in der DE—A—30 30 622 eine oblonge Depottablette vorgeschlagen. Diese besteht aus einem Pressling in länglicher Form, der aus einer gegebenenfalls mit einem Überzug, versehenen, aus mindestens einem Wirkstoff in einer eine verzögerte und kontrollierte Wirkstoffabgabe bewirkenden Hilfsstoffmasse gebildet wird. Dieser Pressling muss bezüglich der geometrischen Abmessungsverhältnisse bestimmte Bedingungen aufweisen.

Die Depottablette DE—A—30 30 622 hat die folgenden Nachteile:

(1) Da bei dieser Retardform de Geschwindigkeit der Wirkstofffreisetzung von Grösse und Art der Gesamtoberfläche abhangig ist un durch Teilung der Tablette die Art und Grösse der Gesamtoberfläche geänder wird, beeinflusst unumgänglich die Tablettenteilung die Wirkstofffreisetzungsgeschwindigkeit der Bruchstücke, wenn auch die Verminderung der Retardwirkung überraschenderweise geringer als erwartet ausfallen soll.

(2) Die Retardform ist an eine bestimmte Form und insbesondere an bestimmte Verhältnisse der geometrischen Abmessungen gebunden. Runde Depottabletten mit Teilkerben sind mit den in der Vorveröffentlichung offenbarten Massnahmen nicht herstellbar.

(3) Die Erfahrung der Tablettenpresstechnologie lehrt, dass insbesondere bei tiefen Kerben aufgrund der unterschiedlichen Verdichtungsverhältnisse in der Nähe der Teilungskerbe der Presslinge eher neben als an der Teilungskerbe bricht. Daher muss bei dieser Ausführungsform des Standes der Technik mit einer schlechten Dosierungsgenauigkeit der Bruchstücke gerechnet werden.

Schliesslich sind auch oral applizierbare Arzneimittelzubereitungen bekannt, bei denen der Wirkstoff in einem Polymeren eingebettet ist. Pharmazeutische Zubereitungen dieser Art werden normalerweise hergestellt, indem man den Wirkstoff zusammen mit einem Polymeren in einem Lösungsmittel auflöst, das Lösungsmittel verdampft und das feste Gemisch granuliert. Normalerweise geschieht die Lösungsmittelentfernung und Granulierung in einem Arbeitsgang durch Sprühtrocknung.

Arzneimittelzubereitungen dieses Typs verfolgen de Zweck, den Wirkstoff feindispers auf dem Polymeren zu verteilen und die Oberfläche des zu lösenden Stoffes zu vergrössern, umd die Auflösung zu beschleunigen und nicht zu verzögern.

Aufgabe der vorliegenden Erfindung ist es, eine teilbare Depottablette zu schaffen, die nicht an bestimmte Verhältnisse der geometrischen Abmessungen gebuden ist und insbesondere keine Abhängigkeit der Wirkstofffreisetzungsgeschwindigkeit von der Grösse und Art der Gesamtoberfläche aufweist. Die erfindungsgemässe Depottablette soll so beschaffen sein, dass die Bruchstücke die gleiche Wirkstofffreisetzungscharakteristik aufweisen wie die ungeteilte Tablette. Erfindungsgemäss wird eine teilbare Tablette mit verzögerter Wirkstofffreigabe auf Polyacrylatbasis vorgeschlagen bei der die Bruchstücke der geteilten Tablette die gleiche Wirkstofffreisetzungscharakteristik aufweisen wie a ungeteilte Tablette, dadurch gekennzeichnet, dass sie aus einem Pressling aus einem emulsionspolymerisierten Polyacrylatmaterial mit einer Korngrösse von 10—550 µm mit im Polymer inkorporierten moleculardispers verteilten Wirkstoff und üblichen Tablettierhilfsstoffen besteht.

Als Polyacrylatmaterial wird ein Copolymerisat von Methyl- und/oder Ethylestern der Acryl- und Methacrylsäure verwendet. Das Copolymerisat soll ein durchschnittliches Molekulargewicht von etwa 800 000 haben.

Es kommt besonders darauf an, ein in bestimmter Weise polymerisiertes Acrylat-Material zu verwenden. Das Acrylat-Copolymerisat muss durch Emulsionspolymerisation hergestellt sein. Es soll zweckmässigerweise eine Teilchengrösse von ungefähr 140 nm haben. Auf andere Weise hergestellt Polyacrylate, z.B. lösungspolymerisierte oder blockpolymerisierte, sind für den erfindungsgemässen Zweck nicht geeignet. Die Gewinnung des Feststoffes aus der Emulsion kann durch Gefriertrocknung oder andere Trocknungsmethoden erfolgen. Hierbei bleiben die Polymerisatteilchen in ihrer Form und Grösse erhalten.

Ein geeignetes Ausgangsmaterial ist das auf dem Markt erhältliche Produkt Eudragit® E30D der Firma Röhm GmbH, Darmstadt. Natürlich können auch andere Polymer-Latices verwendet werden, die bezüglich des Molekulargewichts und der Teilchengrösse den vorbenannten Produkten entsprechen und toxikologisch unbedenklich sind.

Zur Variierung der Freisetzungsgeschwindigkeit kann das Copolymerisat gegebenenfalls saure oder basische Gruppen, z.B. unveresterte Carboxygruppen oder N,N-Dimethylaminoethylestergruppen enthalten. Es ist ferner möglich, Polymerabmischungen zu verwenden, die neben neutralen Poly(meth)acrylestern Ethylenpolymere mit sauren oder basischen Gruppen enthalten, z.B. Poly(meth)acrylsäuren oder Poly-(meth)acrylsäure-N,N-dimethylaminoethylester oder Polyvinylpyrrolidon, die auch ihrerseits als Copolymerisat vorliegen können. Ein Säuregruppen enthaltendes Polymerisat ist z.B. das Handelsprodukt Eudragit L100, das ein Copolymerisat aus Methacrylsäure und Methacrylsäuremethylester mit einem Molekulargewicht von etwa 250 000 darstellt. Ein basische Gruppen enthaltendes Material ist Eudragit E100 der Firma Röhm GmbH, Darmstadt.

Als pharmazeutische Wirkstoffe können erfindungsgemäss saure, basische und neutrale Substanzen mie einem Molekulargewicht bis etwa 500 verwendet werden. Die Wirkstoffe sollen in niedriger Dosierung wirksam sein. In dieser Beziehung kommen insbesondere Wirkstoffe in Betracht, die normalerweise in einer Dosierung bis 50 mg je Gesamttablette angewandt werden. Das Gewicht der Einzeltabletten is nicht kritisch. Normalerweise sollte eine Tablette nicht mehr als 1000 mg weigen.

Um eine verzögerte Wirkstofffreisetzung zu gewährleisten, sollen die im Polyacrylatmaterial eingebetteten Wirkstoffe Diffusionskoeffizienten von $10^{-5}$ bis $10^{-7} cm^2 . h^{-1}$ haben.

Neben dem Diffusionskoeffizienten wird die Freisetzungsgeschwindigkeit auch durch die Teilchengrösse der mit molekulardispers verteiltem Wirkstoff beladenen Polyacrylatteilchen und deren Korngrössenverteilung bestimmt. Zweckmässigerweise liegt die Teilchengrösse zwischen 10 und 500 µm. Kleinere Korngrössen ergeben normalerweise eine höhere Freisetzungsgeschwindigkeit, so dass durch Fraktionierung der Korngrössen eine Variation der Freisetzungsgeschwindigkeit vorgenommen werden kann. Umgekehrt kann man verschiedene Korngrössenfraktionen miteinander mischen, um geeignete Freisetzungsraten zu erzielen.

Die Freisetzungsgeschwindigkeit ist darüber hinaus bei Verwendung saurer oder basischer Arzneimittelwirkstoffe dadurch steuerbar, dass man ein Polyacrylatmaterial verwendet, dass saure oder basische Gruppen enthält. Durch Salsbildung zwischen einem basischen Wirkstoff und im Polyacrylatmaterial enthaltenen sauren Gruppen bzw. zwischen einem sauren Wirkstoff und im Polyacrylatmaterial enthaltenen basischen Gruppen lässt sich die Freigabegeschwindigkeit vermindern.

Schliesslich kann die Freigabegeschwindigkeit durch diffusionsbeeinflussende Stoffe variiert werden, z.B. Polyvinlypyrrolidon, Celluloseester.

Ein weiterer Gegenstand der Erfindung ist ein Verfahren zur Herstellung der oben charakterisierten teilbaren Tablette mit verzögerter Wirkstofffreigabe.

Die Herstellung des für die Tablettierung benötigten feinteiligen Emulsionspolyacrylmaterials mit im Polymeren molekulardispers verteilten Wirkstoff muss in besonderer Weise und in einem getrennten Schritt erfolgen. Es konnte insbesondere festgestellt werden, dass der Versuch, eine Lösung aus dem Arzneimittelwirkstoff und Polyacrylat in einem organischen Lösungsmittel direkt mit einem tablettierbaren Träger zu granulieren, nicht zum Erfolg führt. Die Friegabegeschwindigkeit der aus diesem Granulat hergestellten. Tabletten lag bei etwa 90% des eingesetzen Wirkstoffes in 15 Minuten. Dieser Befund zeigt an, dass bei dem Granulier- und dem anschliessenden Trocknungsprozess der Arzneimittelwirkstoff mit dem Polymerträger auf der angebotenen Oberfläche des Tablettierhilfsstoffes zu stark zerläuft, d.h. der Arzneimittelwirkstoff nicht im Polymeren inkorporiert bzw. gebunden wird.

Die Herstellung der teilbaren Tablette mit verzögerter Wirkstofffreigabe Emulsionspolyacrylmaterial erfolgt in der Weise, dass man den Arzneimittelwirkstoff zusammen mit dem Emulsionspolyacrylmaterial in einem organischen Lösungsmittel löst, das Lösungsmittel abdampft, das feste wirkstoffhaltige. Polyacrylat bei einer unter der Glastemperatur liegenden Temperatur vermahlt, das wirkstoffhaltige Polyacrylatpulver mit üblichen Tablettierhilfsstoffen verarbeitet und das Granulat zu Tabletten verpresst, die gegebenenfalls eine Teilungskerbe enthalten.

Als Lösungsmittel für die Wirkstoff und Polyacrylat enthaltende Lösung kommen insbesondere leicht verdampfbare Solventien in Frage, z.B. Methanol, Ethanol, Aceton, Methylenchlorid, Essigsäuremethyl- und -ethylester, Acetessigsäuremethyl- und -ethylester oder Ether wie Tetrahydrofuran oder Dioxan oder Dimethylsulfoxid bzw. auch Gemische dieser Lösungsmittel.

Um eine schnelle Verdampfung bei möglichst niedriger Temperatur zu gewährleisten, kann die wirkstoff- und polyacrylathaltige Lösung in flache Formen, z.B. Schalen gegossen werden, so dass eine relativ geringe Schichtdicke resultiert. Nach der Trocknung entsteht dann ein Film, dessen Längen- und Dickenabmessungen von dem verwendeten Verdampfungsgefäss abhängen.

Das auf diese Weise erhaltene Polyacrlatmaterial mit dem inkoporierten molekulardispers verteilten Wirkstoff weist eine gewisse Zähigkeit und Eigenklebrigkeit auf un kann als solches nicht vermahlen werden. Zur Zerkleinerung des Polymeren muss es auf eine Temperatur unterhalb der Glastemperatur gebracht, d.h. gekühlt werden. Die Kühlung kann z.B. mit Hilfe von festem oder flüssigem Kohlendioxid, flüssiger Luft oder flüssigem Stickstoff oder anderen verflüssigten inerten Gasen erfolgen. Das Polymere wird dadurch spröde und kann nunmehr zerschlagen werden. Das gemahlene Gut neigt bei Erwärmen auf Zimmertemperatur dazu, sich wieder zusammenzuballen. Daher ist es zweckmässig, dem Mahlgut beim Mahlprozess einen feinteiligen inerten Füllstoff beizufügen, wie etwa Milchzucker, feinteilige Kieselsäure, Magnesiumstearat oder ähnliche feinteilige Gleitmittel. Das Massenverhältnis von wirkstoffhaltigem Polyacrylat zu feinteiligem Füllstoff kann innerhalb weiter Grenzen schwanken und bei etwa 10:1 bis 1:4 liegen.

Auf diese Weise entsteht ein freifliessendes Pulver, das in einem weiteren Schritt mit den üblichen Tablettierhilfsstoffen auf einer normalen Tablettenpresse mit Kerbeinrichtung zu einer teilbaren Tablette verpresst werden kann. Als Tablettierhilfsstoffe kommen insbesondere Bindemittel, inerte Füllstoffe, Gleit- und Schmiermittel sowie Sprengmittel in Frage.

### Beispiel 1

In einem geeigneten Gefäss werden 40 g Eudragit E30D (gefriergetrocknet) 5 g Clonidin-Base und 455 g Aceton zusammengegeben und bis zur vollkommenen Klarheit der Lösung gerührt. Die erhaltene Lösung wird in eine entsprechende Schale überführt, aus der das Lösungsmittel bei Zimmertemperatur abdampfen kann. Der zurückbleibende wirkstoffbeladene Film wird mittels Trockeneis auf etwa −40°C heruntergekühlt und zusammen mit Lactose im Massenverhältnis 9:1 in einer Mühle mit rotierendem Messer unter Kühlung portionsweise gemahlen. Man erhält ein freifliessendes Pulver, in dem das wirkstoffbeladene Polyacrylat in einer Korngrössenverteilung von etwa 10 bis 500 μm vorliegt.

Das in der oben beschriebenen Weise erhaltene Gemisch aus in Polyacrylatkörnern moleculardispers eingebettetem Clonidin und Milchzucker wird im nächsten Arbeitsschritt mit einem weiteren Anteil an Milchzucker und Polyvinylpyrrolidon unter Verwendung von Wasser als Granulationsflüssigkeit granuliert und unter Zusatz der Tablettenhilfsstoffe Maisstärke, kolloidale Kieselsäure und Magnesiumstearat in einer Exzenterpresse mit Kerbeinrichtung zu Tabletten mit einem Gesamtgewicht von 180,0 mg verpresst. Beid diesen Arbeitsschritten werden keine Schwierigkeiten beobachtet. Die genaue Zusammensetzung sowie einige technische Daten der Tablette sind nachstehend aufgeführt:

Tabelle 1

| Bestandteile | Menge in mg/Tablette |
|---|---|
| Polyacrylatträger * | 2,50 |
| Milchzucker | 158,75 |
| Polyvinylpyrrolidon | 3,75 |
| Maisstärke | 13,50 |
| Kolloidale Kieselsäure | 1,00 |
| Magnesiumstearat | 0,50 |
| | 180,00 |

| * Polyacrylatträger | mg/2,50 mg Träger |
|---|---|
| Clonidin-Base | 0,25 |
| Polyacrylat | 2,00 |
| Milchzucker | 0,25 |
| Bruchfestigkeit: | 2,16 N/mm$^2$ |
| Zerfall: | 61 sec |

Die in vitro-Freigabe der Tablette wurde in einer USP-Apparatur bei 37°C durchgeführt, wobei pro Behälter jeweils 4 Tabletten geprüft wurden. Die Umsetzung von Magen- auf Darmsaft erfolgte nach 1 Stunde, die analytische Bestimmung der freigesetzten Clonidin-Base erfolgte mittels Hochdruckflüssigchromatographie. Nach dem Zerfall der Tablette verbleiben neben den wasserunlöslichen Tablettierhilfsstoffen die mit Arzneistoff beladenen Polyacrylatkörnchen zurück, aus denen über den gesamten Beobachtungszeitraum die Clonidin-Base herausdiffundiert.

Freigabewerte:

| Zeit in h | Freigegebene Menge an Clonidin in % |
|---|---|
| 0,25 | 29,1 |
| 1 | 39,5 |
| 2 | 54,9 |
| 4 | 60,8 |
| 6 | 68,3 |

### Beispiel 2

In einem geeigneten Gefäss wurde 38 g Eudragit E30D (gefriergetrocknet) in 430 ml Aceton gelöst und anschliessend mit der Wirkstofflösung, bestehend aus 2 g Effortil-Base® und 80 ml Methanol, versetzt. Die erhaltene, klare Lösung wird in eine Schale überführt, das Lösungsmittel bei Zimmertemperatur abgedampft; es entsteht ein klarer wirkstoffbeladener Film. Der Film wird mit Trockeneis auf ca. −40°C gekühlt und mit Milchzucker im Massenverhältnis 10:1 in einer Mühle zerkleinert und zu Tabletten verarbeitet.

Freigabewerte:

| Zeit in h | Freigegebene Menge in % |
|-----------|-------------------------|
| 0,25      | 10,9                    |
| 1         | 22,9                    |
| 2         | 37,0                    |
| 4         | 46,0                    |
| 6         | 51,5                    |

Die in vitro-Freigabe wurde wie in Beispiel 1 in einer USP-Apparatur durchgeführt. Die analytische Bestimmung des freigesetzten Wirkstoffes erfolgte durch UV-Messung.

### Beispiel 3

In einem geeigneten Gefäss wurden 100 g Eudragit E30D (gefriergetrocknet), 2,5 g Clonidin-Base und 800 g Aceton vorgelegt und so lange gerührt, bis eine klare Lösung resultiert. In einem zweiten Gefäss wurde 25 g Eudragit L 100 in 500 g Ethanol gelöst und zu oder oben genannten Lösung unter Rühren hinzugefügt, wobei eine milchige, weisse Lösung entsteht. Nach Abdampfen des Lösungsmittelgemisches in einer entsprechenden Schale erhält man einen leicht milchigen Film, der nach Kühlung mittels Trockeneis bei ca. −40°C gemahlen und anschliessend zu Tabletten verarbeitet wird.

Freigabewerte:

| Zeit in h | Freigegebene Menge an Clonidin in % |
|-----------|-------------------------------------|
| 0,25      | 11,1                                |
| 1         | 17,1                                |
| 2         | 37,2                                |
| 4         | 41,5                                |
| 6         | 43,5                                |

Die Bestimmung der Freigabewerte erfolgte wie in Beispiel 1 beschrieben.

### Beispiel 4

In einem geeigneten Gefäss wurden 50 g Eudragit E30D (gefriergetrocknet), 2,33 g Clonidin-Base und 5,82 g Polyvinylpyrrolidon vorgelegt, mit 1000 ml Aceton versetzt und unter Rühren gelöst. Nach dem Lösungsvorgang wird das Lösungsmittel in einer offenen Schale abgedampft, der erhaltene Film unter Zusatz von Trockeneis bei ca. −40°C gemahlen und zu Tabletten verarbeitet.

Die Freigabekurve ergibt folgenden prozentualen Werteverlauf:

| Zeit in h | Freigegebene Menge an Clonidin in % |
|-----------|-------------------------------------|
| 0,25      | 12,6                                |
| 1         | 24,7                                |
| 2         | 33,3                                |
| 4         | 44,2                                |
| 6         | 52,5                                |

### Patentansprüche

1. Teilbare Tablette mit verzögerter Wirkstofffreigabe auf Polyacrylatbasis, bei der die Bruchstücke der geteilten Tablette die gleiche Wirkstoffreisetzungscharakterisitik aufweisen wie die ungeteilte Tablette, dadurch gekennzeichnet, daß sie aus einem Pressling aus einem emulsionspolymerisierten Polyacrylatmaterial mit einer Korngröße von 10—500 µm mit in Polymer inkorprierten moleculardispers verteilten Wirkstoff und üblichen Tablettierhilfsstoffen besteht.

2. Tablette nach Anspruch 1, dadurch gekennzeichnet, dass das Polyacrylatmaterial aus einem Copolymerisat von Methyl- und/oder Ethylestern der Acryl- und Methacrylsäure besteht.

3. Tablette nach Anspruch 2, dadurch gekennzeichnet, dass das durchschnittliche Molekulargewicht des Polyacrylatmaterials etwa 800 000 beträgt.

4. Tablette nach einem oder mehreren der vorhergehenden Ansprüche, dadurch gekennzeichnet, dass dem Polyacrylatmaterial Ethylenpolymere mit sauren oder basischen Gruppen beigemischt sind.

5. Tablette nach einem der vorhergehenden Ansprüche, dadurch gekennzeichnet, dass sie als Wirkstoff eine organische Substanz mit einem Molekulargewicht bis etwa 500 und mit einem Diffusionskoeffizienten von etwa $10^{-5}$ bis $10^{-7} cm^2 . h^{-1}$ in einer Dosierung bis 50 mg enthält.

6. Verfahren zur Herstellung einer teilbaren Tablette mit verzögerter Wirkstofffreigabe nach Anspruch 1 bis 5, dadurch gekennzeichnet, daß man den Arzneimittlewirkstoff zusammen mit dem Emulsionspolyacrylatmaterial in einem organischen Löungsmittel löst, das Lösungsmittel abdampft, das feste wirkstoffhaltige Emulsionspolyacrylat bei einer unter der Glastemperatur liegenden Temperatur vermahlt, das wirkstoffhaltige Emulsionpolyacrylatpulver mit üblichen Tablettierhilfsstoffen verarbeitet und das Granulat zu Tabletten verpreßt.

7. Verfahren nach Anspruch 6, dadurch gekennzeichnet, dass die Kühlung des Mahlgutes mit Hilfe von festem oder flüssigem Kohlendioxid, flüssiger Laft flüssigem Stickstoff oder anderen verflüssigten inerten Gasen erfolgt.

8. Verfahren nach Anspruch 5 und/oder 7, dadurch gekennzeichnet, dass man dem Mahlgut einen inerten Füllstoff beifügt.

9. Verfahren nach Anspruch 8, dadurch gekennzeichnet, dass man als Füllstoff Milchzucker, feinteilige Kieselsäure, Magnesiumstearat oder ähnliche feinteilige Gleitmittel verwendet.

### Revendications

1. Pastille divisible à libération retardée de substance active à base de polyacrylate, dans laquelle les fractions de la pastille divisée présentent la même caractéristique de libération de substance active que la pastille non divisée, caractérisée en ce qu'elle consise en un comprimé obtenu à partir d'une matière polyacrylate polymérisée en émulsion ayant une granulométrie de 10—500 µm avec une substance active répartie en dispersion moléculaire incorporée dans le polymère et à partir d'adjuvants usuels pour comprimés.

2. Pastille selon la revendication 1, caractérisée en ce que la matière polyacrylate est constituée par un copolymérisat d'esters de méthyle et/ou d'éthyle de l'acide acrylique et méthyacrylique.

3. Pastille selon la revendication 1, caractérisée en ce que le poids moléculaire moyen de la matière polyacrylate est d'environ 800 000.

4. Pastille selon l'une pou plusieurs des revendications précédentes, caractérisée en ce que l'on ajoute à la matière polyacrylate des polymères d'éthylène comportant des groupes acides ou basiques.

5. Pastille selon l'une des revendications précédents, caractérisée en ce qu'elle contient en tant que substance active, une substance organique ayant un poids moléculaire allant jusqu'à environ 500 et un coefficient de diffusion d'environ $10^{-5}$ jusqu'à $10^{-7} cm^{-2}.h^{-1}$ en un dosage allant jusqu'à 50 mg.

6. Procédé pour la fabrication d'une pastille divisible avec libération retardée de la substance active selon les revendications 1 à 5, caractérisé en ce que l'on dissout la substance active médicamenteuse avec la matière polyacrylate en émulsion dans un solvant organique, en ce que l'on chasse le solvant par évaporation, en ce que l'on broie le polyacrylate solide en émulsion contenant la substance active à une température située au-dessous de la température vitreuse, en ce que l'on transforme la poudre de polyacrylate en émulsion contenant la substance active avec des adjuvants usuels pour comprimés et en ce que l'on forme les pastilles par compression des granulés.

7. Procédé selon la revendication 6, caractérisé en ce que le refroidissement du produit broyé s'effectue à l'aide d'anhydride carbonique solide ou liquide, d'air liquide, d'azote liquide ou d'autres gaz inertes liquéfiés.

8. Procédé selon la revendication 6 et/ou 7, caractérisé en ce que l'on ajoute au produit broyé une matière de charge inerte.

9. Procédé selon la revendication 8, caractérisé en ce que l'on utilise en tant que matière de charge, du lactose, de l'acide silicique finement divisé, du stéarate de magnésium ou des lubrifiants similaires finement divisés.

## Claims

1. Divisible tablet with delayed release of active substance based on polyacrylate, wherein the fragments of the divided tablet have the same characteristics of release of the active substance as the undivided tablet, characterised in that it consists of a compressed blank of an emulsion-polymerised polyacrylate material with a particle size of 10—500 µm having an active substance in molecular dispersion and conventional tablet-making excipients incorporated in the polymer.

2. Tablet as claimed in claim 1, characterized in that the polyacrylate material consists of a copolymer of methyl and/or ethyl esters of acrylic and methyacrylic acid.

3. Tablet as claimed in claim 2, characterized in that the average molecular weight of the polyacrylate material is about 800,000.

4. Tablet as claimed in one or more of the preceding claims, characterized in that the polyacrylate material is mixed with ethylene polymers with acidic or basic groups.

5. Tablet according to one of the preceding claims, characterized in that it contains, as active substance, an organic substance with a molecular weight of up to about 500 and a diffusion coefficient of about $10^{-5}$ to $10^{-7}.h^{-1}$ in a dosage up to 50 mg.

6. Process for preparing a divisible tablet with delayed release of active substance according to claims 1 to 5, characterized in that the pharmaceutically active substance together with the emulsion polyacrylate material is dissolved in an organic solvent, the solvent is evaporated off, the solid emulsion polyacrylate containing the active substance is ground at a temperature below the glass transition temperature, the emulsion polyacrylate powder containing the active substance is processed with conventional tablet-making excipients and the granulated material is compressed to form tablets.

7. Process as claimed in claim 6, characterized in that the ground material is cooled with the aid of solid or liquid carbon dioxide, liquid air, liquid nitrogen, or other liquefied inert gases.

8. Process as claimed in claim 6 and/or 7, characterized in that an inert filler is added to the ground material.

9. Process as claimed in claim 8, characterized in that lactose, finely divided silica, magnesium-stearate or other finely divided lubricants are used as a filler.